# EUROPÄISCHE PATENTSCHRIFT

(11) **EP 1 615 869 B1**
(45) Veröffentlichungstag und Bekanntmachung des Hinweises auf die Patenterteilung: **29.11.2006**
(21) Anmeldenummer: 04726984.0
(22) Anmeldetag: 13.04.2004
(51) Int. Cl.: C07C 51/44, E04B 1/00

(54) **THERMISCHES TRENNVERFAHREN UND TRENNKOLONNE**
THERMAL RECTIFICATION METHOD, AND RECTIFYING COLUMN
PROCEDE DE SEPARATION THERMIQUE ET COLONNE DE SEPARATION

(30) Priorität: 15.04.2003 DE 10317436; 06.06.2003 US 476161 P
(43) Veröffentlichungstag der Anmeldung: 18.01.2006
(73) Patentinhaber: BASF Aktiengesellschaft, 67056 Ludwigshafen (DE)
(72) Erfinder: DIEHL, Volker, 67158 Ellerstadt (DE); JÄGER, Ulrich, 67354 Römerberg (DE); HAMMON, Ulrich, 68163 Mannheim (DE); SCHRÖDER, Jürgen, 67071 Ludwigshafen (DE); RISSEL, Steffen, 67281 Kirchheim (DE)
(86) Internationale Anmeldenummer: PCT/EP2004/003847
(87) Internationale Veröffentlichungsnummer: WO 2004/092108

(56) Entgegenhaltungen:
- EP-A- 0 937 488
- EP-A- 1 044 957
- GB-A- 514 267
- US-A- 2 267 929
- US-A- 5 788 895
- US-B1- 6 267 359

## Beschreibung

Vorliegende Erfindung betrifft ein thermisches Trennverfahren zwischen wenigstens einem in einer eine Abfolge von Stoffaustauschböden enthaltenden Trennkolonne aufsteigenden gasförmigen und wenigstens einem in der Trennkolonne absteigenden, Polymerisationsinhibitor gelöst enthaltenden, flüssigen Stoffstrom, von denen wenigstens einer (Meth)acrylmonomere enthält und bei dem innere Oberfläche der Trennkolonne mit dem in der Trennkolonne absteigenden, Polymerisationsinhibitor gelöst enthaltenden, flüssigen Stoffstrom besprüht wird und wobei die Trennkolonne Einbauten aufweist, von denen sich wenigstens Teilflächen im Schattenbereich des versprühten absteigenden flüssigen Stoffstroms befinden.

Die Schreibweise (Meth)acrylmonomere steht in dieser Schrift verkürzend für "Acrylmonomere und/oder Methacrylmonomere".

Der Begriff Acrylmonomere steht in dieser Schrift verkürzend für "Acrolein, Acrylsäure und/oder Ester der Acrylsäure".

Der Begriff Methacrylmonomere steht in dieser Schrift verkürzend für "Methacrolein, Methacrylsäure und/oder Ester der Methacrylsäure".

Im besonderen sollen die in dieser Schrift angesprochenen (Meth)acrylmonomeren die nachfolgenden (Meth)acrylsäureester mit umfassen: Methylacrylat, Methylmethacrylat, n-Butylacrylat, iso-Butylacrylat, iso-Butylmethacrylat, n-Butylmethacrylat, tert.-Buylacrylat, tert.-Butylrnethacrylat, Ethylacrylat, Ethylmethacrylat, 2-Ethylhexylacrylat, 2-Ethylhexylmethacrylat, N,N-Dimethylaminoethylacrylat, N,N-Dimethylaminoethylmethacrylat, Cyclohexylmethacrylat, 1,4-Butandiolmonoacrylat, Hydroxyethylacrylat, Hydroxyethylmethacrylat, Hydroxypropylacrylat, Hydroxypropylmethacrylat, Glycidylacrylat und Glycidylmethacrylat.

(Meth)acrylmonomere sind aufgrund ihrer sehr reaktionsfähigen ethylenisch ungesättigten Doppelbindung wertvolle Ausgangsverbindungen zur Herstellung von Polymerisaten, die z.B. als Klebstoff, wasserabsorbierendes Harz oder als Bindemittel für Dispersionsfarben Verwendung finden.

(Meth)acrolein und (Meth)acrylsäure wird großtechnisch überwiegend durch katalytische Gasphasenoxidation geeigneter C₃-/C₄-Vorläuferverbindungen (oder von Vorläuferverbindungen derselben), insbesondere von Propen und Propan im Fall von Acrolein und Acrylsäure bzw. von iso-Buten und iso-Butan im Fall der Methacrylsäure und des Methacroleins, hergestellt. Neben Propen, Propan, iso-Buten und iso-Butan eignen sich als Ausgangsstoffe jedoch auch andere 3 bzw. 4 Kohlenstoffatome enthaltende Verbindungen wie iso-Butanol, n-Propanol oder Vorläuferverbindungen derselben wie z.B. der Methylether von iso-Butanol. (Meth)acrylsäure kann auch aus (Meth)acrolein erzeugt werden.

Dabei wird normalerweise ein Produktgasgemisch erhalten, aus dem die (Meth)acrylsäure bzw. das (Meth)acrolein abgetrennt werden muss.

Ester der (Meth)acrylsäure sind z.B. durch direkte Umsetzung von (Meth)acrylsäure und/oder (Meth)acrolein mit den entsprechenden Alkoholen erhältlich. Allerdings fallen auch in diesem Fall zunächst, in der Regel flüssige, Produktgemische an, aus denen die (Meth)acrylsäureester abgetrennt werden müssen (z.B. rektifikativ).

Für vorstehende Abtrennungen werden häufig Trennverfahren angewendet, die in eine Abfolge von Stoffaustauschböden als trennwirksame Einbauten enthaltenden Trennkolonnen durchgeführt werden. In diesen Trennkolonnen werden vielfach gasförmige (aufsteigend) und flüssige (absteigend) Stoffströme im Gegenstrom geführt, wobei infolge des zwischen den Stoffströmen bestehenden Ungleichgewichts ein Wärme- und Stoffaustausch stattfindet, der letztlich die in der Trennkolonne gewünschte Auftrennung bedingt. In dieser Schrift sollen solche Trennverfahren als thermische Trennverfahren bezeichnet werden.

Beispiele für und damit Element des in dieser Schrift verwendeten Begriffs "thermische Trennverfahren" sind die fraktionierende Kondensation (vgl. DE-A 19924532) und/oder die Rektifikation (aufsteigende Dampfphase wird im Gegenstrom zu absteigender Flüssigphase geführt; die Trennwirkung beruht darauf, dass die Dampfzusammensetzung im Gleichgewicht anders als die Flüssigzusammensetzung ist), die Absorption (wenigstens ein aufsteigendes Gas wird zu wenigstens einer absteigenden Flüssigkeit im Gegenstrom geführt; die Trennwirkung beruht auf der unterschiedlichen Löslichkeit der Gasbestandteile in der Flüssigkeit), die Strippung (wie die Absorption; die Flüssigphase ist jedoch mit einer Komponente beladen, die vom Stippgas aufgenommen wird) und die Desorption (der Umkehrprozess zur Absorption; das in der Flüssigphase gelöste Gas wird durch Partialdruckemiedrigung abgetrennt).

Beispielsweise kann die Abtrennung von (Meth)acrylsäure bzw. (Meth)acrolein aus dem Produktgasgemisch der katalytischen Gasphasenoxidation so durchgeführt werden, dass die (Meth)acrylsäure bzw. das (Meth)acrolein durch Absorption in ein Lösungsmittel (z.B. Wasser oder ein organisches Lösungsmittel) oder durch fraktionierende Kondensation des Produktgasgemisches zunächst grundabgetrennt und das dabei anfallende Kondensat bzw. Absorbat nachfolgend rektifikativ (in der Regel in mehreren Stufen) unter Erhalt von mehr oder weniger reiner (Meth)acrylsäure bzw. (Meth)acrolein aufgetrennt wird (vgl. z.B. EP-A 717019, EP-A 1125912, EP-A 982289, EP-A 982287, DE-A 19606877, DE-A 1011527, DE-A 10224341 und DE-A 10218419).

Die vorstehend angesprochene fraktionierende Kondensation unterscheidet sich von der herkömmlichen Rektifikation im wesentlichen dadurch, dass das aufzutrennende Gemisch der Trennkolonne gasförmig (d.h., vollständig in die Dampfform überführt) zugeführt wird.

Anstelle der fraktionierenden Kondensation kann auch zunächst eine Totalkondensation angewandt und das dabei anfallende Kondensat anschließend rektifikativ aufgetrennt werden.

Die bereits angesprochenen, (Meth)acrylmonomere enthaltenden, gasförmigen bzw. flüssigen Gemische können die (Meth)acrylmonomere sowohl in mehr oder weniger reiner Form als auch in Verdünnung (z.B. mit Lösungsmittel oder mit Verdünnungsgasen) befindlich enthalten. Das Lösungsmittel kann dabei sowohl wässrig als auch ein organisches Lösungsmittel sein, wobei die spezifische Art des organischen Lösungsmittels im wesentlichen unbeachtlich ist. Das Verdünnungsgas kann z.B. Stickstoff, Kohlenoxid (CO, CO₂), Sauerstoff, Kohlenwasserstoff oder ein Gemisch aus diesen Gasen sein.

Das heißt, z.B. auf dem Weg der Gewinnung von (Meth)acrylmonomeren werden auf unterschiedlichste Art und Weise thermische Trennverfahren auf gasförmige und/oder flüssige Stoffgemische angewendet, deren Gehalt an (Meth)acrylmonomeren
≥ 2 Gew.-%, oder ≥ 10 Gew.-%, oder ≥ 20 Gew.-%, oder ≥ 40 Gew.-%, oder
≥ 60 Gew.-%, oder ≥ 80 Gew.-%, oder ≥ 90 Gew.-%, oder ≥ 95 Gew.-%, oder
≥ 99 Gew.-% betragen kann.

Die Anreicherung der (Meth)acrylmonomere kann dabei sowohl am Kopf als auch im Sumpf der Trennkolonne erfolgen. Selbstredend können aber auch im oberen, unteren oder mittleren Teil der Trennkolonne (Meth)acrylmonomere angereichert enthaltende Fraktionen entnommen werden.

Die in den Trennkolonnen für die thermischen Trennverfahren enthaltenen Stoffaustauschböden verfolgen den Zweck, in der Trennkolonne in Form von Flüssigkeitsschichten Orte mit geschlossenen flüssigen Phasen zur Verfügung zu stellen. Die Oberfläche des in der Flüssigkeitsschicht aufsteigenden und sich dabei in der geschlossenen flüssigen Phase verteilenden Dampf- bzw. Gasstromes ist dann die maßgebende Austauschfläche.

Dabei überströmt die Flüssigkeit den Stoffaustauschboden, der eine Vielzahl von Durchtrittsöffnungen aufweist. Durch diese Durchtrittsöffnungen steigt das Gas auf, so dass der Stoffaustauschvorgang stattfinden kann. Die Rücktaufftüssigkeit wird durch dieselben Öffnungen oder durch spezielle Ablaufeinrichtungen (Schächte) von Boden zu Boden weitergeleitet. Letztere fallen üblicherweise nicht unter den Begriff der Durchtrittsöffnung.

Ein Problemfeld bei der Durchführung thermischer Trennverfahren zwischen wenigstens einem gasförmigen und wenigstens einem flüssigen Stoffstrom, von denen wenigstens einer (Meth)acrylmonomere enthält, besteht darin, dass (Meth)acrylmonomere bezüglich ihrer radikalischen Polymerisation sehr reaktiv sind und zu unerwünschter Polymerisation neigen.

Üblicherweise werden die Trennkolonnen daher polymerisationsinhibiert betrieben. D.h., dem in der Trennkolonne absteigenden flüssigen Stoffstrom (in dieser Schrift auch Rücklauf oder Rücklaufflüssigkeit genannt) werden Polymerisationsinhibitoren (z.B. phenolische Verbindungen, Aminoverbindungen, Nitroverbindungen, Phosphorverbindungen, Schwefelverbindungen, N-Oxylverbindungen und/oder Schwermetallsalze) zugesetzt.

Alle Flächen der Trennkolonne, die mit dem in der Trennkolonne absteigenden flüssigen Stoffstrom benetzt werden, sind so automatisch polymerisationsinhibiert.

Bezüglich der dem in der Trennkolonne absteigenden flüssigen Stoffstrom zugewandten Flächen (z.B. die Oberseite der Stoffaustauschböden) ist der vorgenannte Wirkzusammenhang vergleichsweise problemfrei.

Dies trifft jedoch nicht mehr auf diejenigen Flächen der Trennkolonne zu, die dem absteigenden flüssigen Stoffstrom abgewandt sind (z.B. die Unterseite der Stoffaustauschböden).

An ihnen können im aufsteigenden gasförmigen Stoffstrom uninhibiert enthaltene (Meth)acrylmonomere auskondensieren. Das nicht inhibierte Kondensat (in der kondensierten Phase ist die Polymerisationsneigung aufgrund der geringen intermolekularen Abstände besonders ausgeprägt) kann dann polymerisieren, sich dabei ausbildendes Polymerisat aufwachsen und letztendlich den weiteren Betrieb der Trennkolonne unmöglich machen.

Die EP-A 937488 und die EP-A 1044957 beschreiben daher Verfahren der Rektifikation von (Meth)acrylmonomere enthaltenden Stoffgemischen, bei denen die innere Oberfläche der Rektifikationskolonne, einschließlich der Stoffaustauschbodenunterseite, über Düsen mit polymerisationsinhibiertem Rücklauf besprüht wird.

Die DE-A 10300816 betrifft thermische Trennverfahren von (Meth)acrylmonomere enthaltenden Stoffgemischen, bei denen die eine Abfolge von Stoffaustauschböden enthaltende Trennkolonne so betrieben wird, dass der nach oben gerichtete gasförmige Stoffstrom beim Durchtritt durch die Durchtrittsöffnungen der Stoffaustauschböden von der auf selbigen befindlichen polymerisationsinhibierten Flüssigphase kleine Flüssigkeitströpfchen mitreißt und nach oben gerichtet versprüht.

Nachteilig an den Verfahrensweise der EP-A 937488, EP-A 1044957 und DE-A 10300816 ist, dass über sie nur mit erheblichem Aufwand ein flächendeckendes Besprühen mit polymerisationsinhibiertem Rücklauf erreicht werden kann.

D.h., es wird immer wieder Teilflächen von in der Trennkolonne befindlichen Einbauten geben, die weder dem absteigenden polymerisationsinhibierten flüssigen Stoffstrom zugewandt sind, noch von versprühtem polymerisationsinhibiertem Rücklauf in ausreichendem Ausmaß erfasst werden.

Solche Teilflächen sollen in dieser Schrift als Teilflächen bezeichnet werden, die sich im Schattenbereich des versprühten (entweder überspezielle Düsen und/oder über Stoffaustauschböden) absteigenden flüssigen Stoffstroms befinden.

Die Aufgabe der vorliegenden Erfindung bestand nun darin, solche im Schattenbereich von versprühtem polymerisationsinhibiertem Rücklauf befindliche Teilflächen von in für thermische Trennverfahren geeigneten Trennkolonnen enthaltenen Einbauten auf möglichst einfache Art und Weise und ohne erhöhten Aufwand, insbesondere ohne zusätzlich einzusetzende Sprühdüsen, aus dem Schattenbereich herauszuführen.

Demgemäss wurde ein thermisches Trennverfahren zwischen wenigstens einem in einer eine Abfolge von Stoffaustauschböden enthaltenden Trennkolonne aufsteigenden gasförmigen und wenigstens einem in der Trennkolonne absteigenden, Polymerisationsinhibitor gelöst enthaltenden, flüssigen Stoffstrom, von denen wenigstens einer (Meth)acrylmonomere enthält und bei dem innere Oberfläche der Trennkolonne mit dem in der Trennkolonne absteigenden, Polymerisationsinhibitor gelöst enthaltenden, flüssigen Stoffstrom besprüht wird und wobei die Trennkolonne Einbauten aufweist, von denen sich wenigstens Teilflächen im Schattenbereich des versprühten absteigenden flüssigen Stoffstroms befinden, gefunden, das dadurch gekennzeichnet ist, dass im Schatten des versprühten absteigenden flüssigen Stoffstroms befindliche Teilflächen der Einbauten durch Verkleiden aus dem Schattenbereich herausgeführt werden.

Eine Abfolge von Stoffaustauschböden sollen in dieser Schrift wenigstens zwei, besser wenigstens drei oder wenigstens vier aufeinanderfolgende Stoffaustauschböden sein, die durch keine anderen trennwirksamen Einbauten unterbrochen werden.

Häufig wird bei erfindungsgemäßen Verfahren innere Oberfläche der Trennkolonne dadurch mit dem in der Trennkolonne absteigenden flüssigen Stoffstrom besprüht, dass der nach oben gerichtete gasförmige Stoffstrom beim Durchtritt durch Stoffaustauschböden von der auf selbigen befindlichen Flüssigphase kleine Flüssigkeitströpfchen mitreißt und nach oben gerichtet versprüht.

Im Folgenden soll die vorliegende Erfindung ohne Beschränkung ihrer Allgemeinheit an einigen Beispielen erläutert werden.

Häufig enthalten Trennkolonnen für thermische Trennverfahren als Stützelemente Doppel-T-Träger eingebaut, die von einer Seite der Trennkolonne bis zur anderen Seite der Trennkolonne ragen und an der Kolonnenwand befestigt sind.

Im Unterschied zu den einfachen T-Trägem wie sie z.B. die EP-A 759316 zeigt, sind Doppel-T-Träger erhöht tragfähig. Dies ist u.a. damit verknüpft, dass sie zwei Querschenkel aufweisen, wohingegen der einfache T-Träger nur einen Querschenkel aufweist. Der zweite Querschenkel versteift den Längsschenkel zusätzlich.

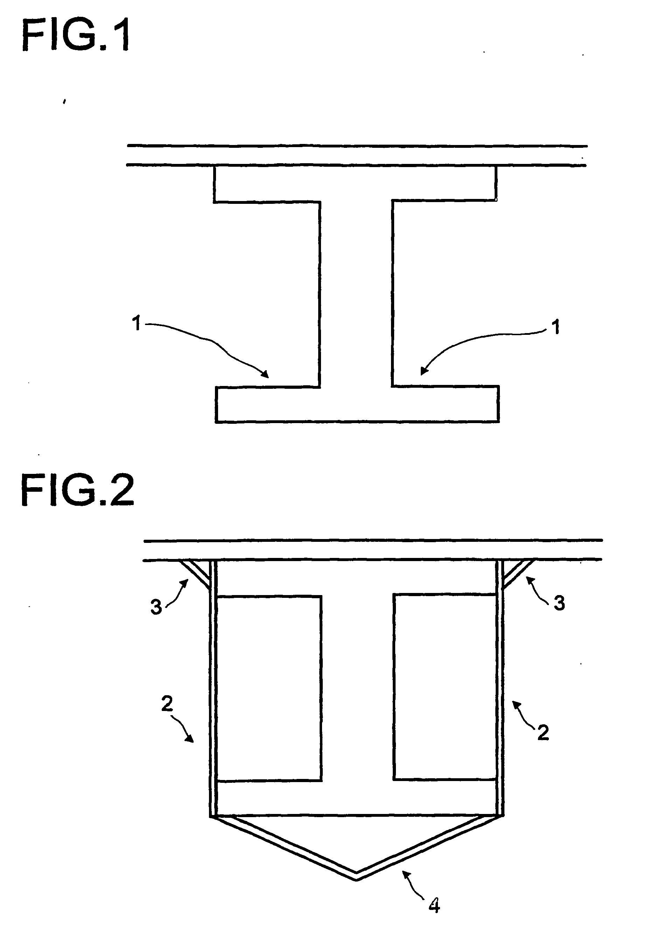

Figur 1 zeigt den Querschnitt eines Doppel-T-Trägers mit aufliegendem Stoffaustauschboden.

Nachteilig an Doppel-T-Trägem ist jedoch, dass z.B. die in Figur 1 mit der Ziffer (1) gekennzeichneten Teilflächen von Doppel-T-Trägem im Sprühschatten der Flüssigkeitströpfchen liegen, die der nach oben gerichtete gasförmige Stoffstrom beim Durchtritt durch die Durchtrittsöffnungen des darunter liegenden Stoffaustauschbodens von der auf selbigem befindlichen Flüssigphase mitreißt und im wesentlichen vertikal nach oben gerichtet versprüht. Figur 2 zeigt, wie diesem Nachteil durch einfaches Anbringen einer Verkleidung abgeholfen werden kann, ohne dass es einer zusätzlichen Sprühdüse für polymerisationsinhibierten Rücklauf bedarf.

Bevorzugt weist die Verkleidung noch die Elemente (3) auf. Sie erleichtern den Ablauf von an der Unterseite des auf dem Doppel-T-Träger aufliegenden Stoffaustauschboden sich ansammelnden polymerisationsinhibierten Flüssigkeitströpfchen, die vom darunterliegenden Stoffaustauschboden nach oben gesprüht wurden. Sich an der vertikalen Verkleidungswand gegebenenfalls ausbildende Polymerisationskeime werden so kontinuierlich weggeschwemmt und in den Kolonnensumpf transportiert. Polymerisationsinhibierung ist gewährleistet.

Erfindungsgemäß (4) günstig ist es ferner, wenn die Verkleidung noch eine Abtropfnase (4) aufweist, da sie die Verweilzeit von ablaufender Rücklaufflüssigkeit mindert. Mit zunehmender Verweilzeit erhöht sich jedoch die Polymerisationswahrscheinlichkeit.

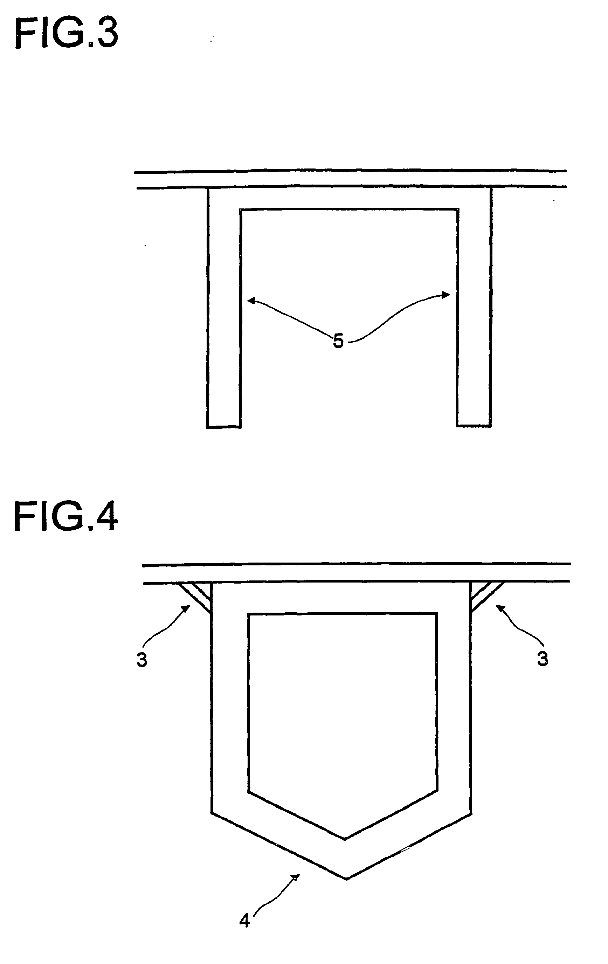

Das gleiche trifft auch auf U-förmige Träger zu (da sie zwei Längsschenkel aufweisen, können die Längsschenkel bei gleicher Tragfähigkeit kürzer sein, was besonders kleine Abstände der Stoffaustauschböden ermöglicht), wie sie die Figur 3 mit aufliegendem Stoffaustauschboden zeigt. Hier bilden beispielsweise die vertikalen Innenwände (5) typische Problemflächen. Figur 4 zeigt die Problemlösung in Form einer eleganten Verkleidung.

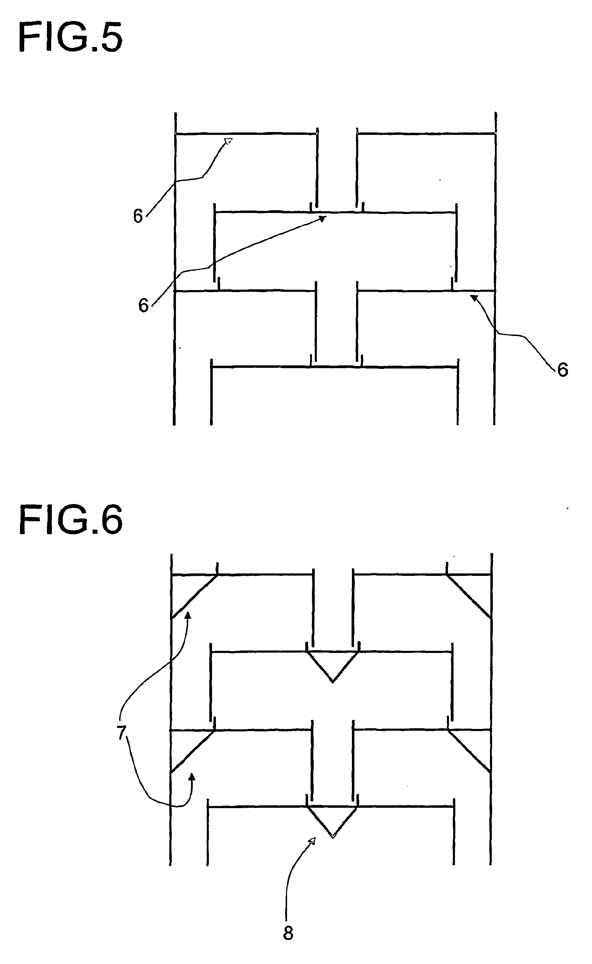

In ähnlicher Weise zeigt Figur 5 eine Abfolge von Stoffaustauschböden, mit altemierend zentriertem und seitlichem Ablaufschacht für die polymerisationsinhibierte Rücklaufflüssigkeit. Dort, wo der Ablaufschacht des unmittelbar darüberliegenden Bodens auf den darunter liegenden Boden mündet, liegt die Unterseite des übernächsten Bodens im Sprühschatten (6), da im Schachtbereich infolge unzureichender Druckverhältnisse normalerweise keine Versprühung erfolgt. Figur 6 zeigt, wie auch hier durch einfaches Verkleiden (7), (8) abgeholfen werden kann. Sowohl die Verkleidung (7) als auch die Verkleidung (8) gewährleistet den gerichteten Ablauf von auf der übrigen Bodenunterseite sich ansammelnder Flüssigkeitströpfchen, die vom gasförmigen Durchtritt durch die Durchtrittsöffnungen des darunter liegenden Stoffaustauschbodens von der auf selbigem befindlichen Flüssigkeitsphase mitgerissen werden.

Die für das erfindungsgemäße Verfahren geeigneten Trennkolonnen können Stoffaustauschböden der unterschiedlichsten Art enthalten. Neben Stoffaustauschböden können die für das erfindungsgemäße Verfahren geeigneten Trennkolonnen als trennwirksame Einbauten aber auch zusätzlich z.B. Packungen oder Füllkörper enthalten.

Erfindungsgemäß bevorzugt enthalten die für das erfindungsgemäße Verfahren geeigneten Trennkolonnen als trennwirksame Einbauten nur Stoffaustauschböden.

Für die Durchtrittsöffnungen der für das erfindungsgemäße Verfahren geeigneten Stoffaustauschböden ist eine Vielzahl von Gestaltungsmöglichkeiten bekannt. Es können im wesentlichen plane Öffnungen vorgesehen sein (Siebböden), die Öffnungen können mit Ventilen versehen sein (Ventilböden) und die Öffnungen können auch mit Glocken gegen die Rücklaufflüssigkeit abgeschirmt sein (Glockenböden). Erfindungsgemäß geeignet sind auch höher integrierte Stoffaustauschbodengestaltungen wie Tunnel- und Zentrifugatböden, bei denen immer mehrere Durchtrittsöffnungen zu einer Gruppe von Gasdurchtritten zusammengefasst sind und bei denen die Richtung des Flüssigkeitsstromes durch einen Impuls des durch-/ausströmenden gasförmigen Stoffstroms gesteuert wird.

Im übrigen sind bei den heute verwendeten Trennkolonnen mit Durchmesser von mehreren Metern die Stoffaustauschböden aus mehreren Planen zusammengesetzt, die auf oder an Trägern befestigt sind, wie es z.B. die EP-A 759316 beschreibt.

Ein Klassiker untern den Stoffaustauschböden und für das erfindungsgemäße Verfahren in besonderem Maß geeignet ist der Siebboden. Darunter sollen in dieser Schrift Platten verstanden werden, die als Durchtrittsstellen für die aufsteigenden Gas- bzw. Dampfströme (die Begriffe "gasförmig" und "dampfförmig" werden in dieser Schrift synonym verwendet) einfache Löcher und/oder Schlitze aufweisen.

Die Siebböden werden dabei in zwei Gruppen differenziert, nämlich in solche mit Flüssigkeitszwangsführung und solche ohne Flüssigkeitszwangsführung.

Die Zwangsführung der Rücklaufflüssigkeit wird dabei dadurch erzielt, dass die Siebböden neben den Durchtrittsstellen für die aufsteigenden Gas- bzw. Dampfströme wenigstens einen Ablaufschacht (Ablauf) aufweisen, durch den die Rücklaufflüssigkeit unabhängig vom Strömungsweg des Gas- bzw. Dampfstroms vom höher gelegenen Boden auf den nächsten tiefer gelegenen Boden fließt (Zulauf). Die Rücklaufflüssigkeit im Querstrom über den Boden vom wenigstens einen Zulauf zum wenigstens einen Ablauf, wobei das Zulauf- und Ablaufrohr den Flüssigkeitsverschluss und die gewünschte Flüssigkeitshöhe auf dem Boden garantieren.

Häufig (insbesondere bei geringen Kolonnendurchmessem) sind die Siebböden mit Flüssigkeftszwangsführung einflutig gestaltet. Ganz allgemein wird auf Stoffaustauschböden Flüssigkeitszwangsführung dadurch erzielt, dass die Stoffaustauschböden wenigstens einen Ablaufschacht (Ablauf) aufweisen, durch den die Rücklaufflüssigkeit unabhängig vom Strömungsweg des aufsteigenden Dampfstroms vom höher gelegenen Boden auf den tiefer gelegenen Boden fließt (Zulauf). Die horizontale Flüssigkeitsströmung über den Stoffaustauschboden vom Zulauf zum Ablauf wird entsprechend der verfahrenstechnischen Aufgabenstellung gewählt. Der aufsteigende Gasstrom tritt durch die Durchtrittsöffnungen des Stoffaustauschbodens. Wird die Rücklaufflüssigkeit im Umkehrstrom über den Stoffaustauschboden geführt (Zulauf und Ablauf des Stoffaustauschbodens sind auf der gleichen Seite des Stoffaustauschboden angeordnet), spricht man von Umkehrstromböden.

Bei Radialstromböden strömt die Flüssigkeit auf dem Stoffaustauschboden radial von der Mittel (Zulauf) zum Ablauf am Rand des Bodens.

Bei den Querstromböden wird die Flüssigkeit über den gesamten Fließbereich betrachtet quer über den Boden vom Zulauf zum Ablauf geführt. In der Regel sind Querstromböden einflutig gestaltet. D.h., Zulauf und Ablauf sind auf gegenüberliegenden Seiten des Stoffaustauschbodens angeordnet. Sie können aber auch zweiflutig (oder auch mehr als zweiflutig) gestaltet sein.

In diesem Fall kann der Zulauf z.B. in der Mitte und je ein Ablauf auf gegenüberliegenden Seiten des Stoffaustauschbodens angeordnet sein.

Bevorzugt sind Querstromsiebböden.

D.h., Zulauf und Ablauf sind auf gegenüberliegenden Seiten des Stoffaustauschbodens angeordnet. Sie können aber auch zweiflutig (oder auch mehr als zweiflutig) gestaltet sein. In diesem Fall kann der Zulauf z.B. in der Mitte und je ein Ablauf auf gegenüberliegenden Seiten des Stoffaustauschbodens angeordnet sein. In dieser Schrift sollen solche Siebböden als Zwangssiebböden bezeichnet werden.

Bei ihnen wird ein die Trennwirkung minderndes Durchregnen der Rücklaufflüssigkeit nicht wie beim Glockenboden durch Kamine verhindert, in die die Durchtrittsöffnungen fortführen, sondern es bedarf dazu einer minimalen Dampfbelastung. Der Dampf tritt aufsteigend durch die Durchtrittsöffnungen und durchperlt die vom Ablaufrohr gehaltene Flüssigkeitsschicht.

Von den Zwangssiebböden unterscheiden sich die Dual-Flow oder auch Regensiebböden dadurch, dass die kein Ablaufsegment enthalten. Durch die Abwesenheit von Ablaufsegmenten (Ablauf schächten) treten bei den Regensiebböden der aufsteigende gasförmige Stoffstrom und der in der Trennkolonne absteigende flüssige Stoffstrom durch die gleichen Durchtrittsstellen des Bodens. Auch beim Regensiebboden bedarf es wie beim Zwangssiebboden einer minimalen Dampfbelastung, um eine angemessene Trennwirkung zu erzielen. Wird sie signifikant unterschritten, bewegen sich aufsteigendes Gas und absteigender Rücklauf im wesentlichen ohne Austausch aneinander vorbei und der Stoffaustauschboden läuft Gefahr, trocken zu laufen. D.h., auch beim Regensiebboden muss eine untere Grenzgeschwindigkeit vorhanden sein, damit auf dem Boden eine gewisse Flüssigkeitsschicht gehalten wird, um ein Arbeiten des Bodens zu ermöglichen.

Von den Querstromsiebböden unterscheiden sich die hydraulisch abgedichteten Querstromböden dadurch, dass sie beim Abschalten der Trennkolonne nicht leer laufen können, sieht man von der winzigen Leerlaufbohrung (ihr Querschnitt ist normalerweise mehr als 200 mal kleiner als der Gesamtquerschnitt) ab, die jeder Querstromboden aus Zweckmäßigkeitsgründen aufweist.

D.h., auch bei geringen Belastungen der Trennkolonne weisen hydraulisch abgedichtete Querstromböden gestaute Flüssigkeit auf und laufen keine Gefahr, trocken zu laufen. Dies ist dadurch bedingt, dass es sich bei den Durchtrittsstellen von hydraulisch abgedichteten Querstromböden nicht wie z.B. bei Dual-Flow-Böden, Siebböden und Ventilböden um kaminlose Bohrungen handelt. Vielmehr mündet jede Durchtrittsstelle in einen Kamin, der ein Trockenlaufen unterbindet. Über dem Kamin sind Dampfumlenkhauben (Glocken) angebracht, die in die gestaute Bodenflüssigkeit eintauchen. Häufig sind die Dampfumlenkhauben an ihren Rändern geschlitzt oder gezackt (d.h., sie weisen Treibschlitze auf). Der durch die Durchtrittsstelle aufsteigende Dampfstrom erfährt durch die Dampfumlenkhauben eine Ablenkung und strömt parallel zum Boden, d.h., quer zur Kolonne in die gestaute Flüssigkeit.

Die aus benachbarten, in der Regel über den Boden äquidistant verteilt angeordneten, Hauben austretenden Dampfblasen bilden in der gestauten Flüssigkeit eine Sprudelschicht aus.

Ablaufrohre bzw. -segmente, die, in die Regel abwechselnd links oder rechts, die Böden verlassen, regeln - von Wehren unterstützt - den Flüssigkeitsstand der Stoffaustauschböden und führen die Flüssigkeit dem darunter liegenden Boden zu.

Für die hydraulisch abdichtende Wirkung ist wesentlich, dass die Ablaufrohre bzw.-segmente des oberen Bodens in die gestaute Flüssigkeit des darunter liegenden Bodens tauchen. Vorzugsweise sind keine Zulaufwehre vorhanden. In der Höhe einstellbare Glocken gestatten ein Anpassen an die Strömungsverhältnisse und den Ausgleich der Eintauchtiefen bei Herstellungsungleichmäßigkeiten, so dass alle Glocken des Bodens gleichmäßig gasen.

Je nach Gestalt und Anordnung der Glocken unterscheidet man z.B. die einflutig gestalteten hydraulisch abgedichteten Querstromböden in Rundglockenböden (Durchtrittsstelle, Kamin und Glocke sind rund), Tunnel-Böden (Durchtnttsstelte, Kamin und Glocke sind rechteckig, die Glocken sind hintereinander angeordnet, wobei die längere Rechteckkante parallel zur Querstromrichtung der Flüssigkeit ausgerichtet ist) und Thormann-Böden (Durchtrittsstelle, Kamin und Glocke sind rechteckig, die Glocken sind hintereinander angeordnet, wobei die längere Rechteckkante senkrecht zur Querstromrichtung der Flüssigkeit ausgerichtet ist. Modifizierte Thormann-Böden sind in der DE-A 10243625 beschrieben.

Siebböden unterscheiden sich dadurch von hydraulisch abgedichteten Querstromböden, dass bei ihnen durch die stets nach oben gerichtete Strömungsrichtung des Dampfes die Neigung zum Mitreißen kleiner Flüssigkeitstropfen erhöht, ihre Neigung zum Versprühen von Rücklaufflüssigkeit ausgeprägter ist.

Das erfindungsgemäße Verfahren eignet sich deshalb insbesondere für solche Trennkolonnen, die als Stoffaustauschböden Siebböden enthalten. Besonders bevorzugt enthalten sie als trennwirksame Einbauten ausschließlich Siebböden. Bevorzugt sind innerhalb der Siebböden dabei die Regensiebböden. Selbstverständlich können die beim erfindungsgemäßen Verfahren verwendeten Trennkolonnen auch andere Stoffaustauschböden, z.B. Ventilböden und/oder hydraulisch abgedichtete Querstromböden, enthalten. Letztere können in der Trennkolonne auch gemeinsam mit Siebböden und/oder anderen trennwirksamen Einbauten enthalten sein.

Wird der Gewichtsanteil der gesamten in einer in erfindungsgemäßem Betrieb befindlichen Trennkolonne einem Stoffaustauschboden zugeführten Flüssigkeitsmenge, die durch den aufsteigenden Gasstrom bis zur Unterseite des nächst höher gelegenen Stoffaustauschbodens mitgerissen wird als Mitrissanteil (in Gew.-%) dieses Stoffaustauschbodens bezeichnet (zur Definition und experimentellen Bestimmung siehe die DE-A 10300816, so wird das erfindungsgemäße Verfahren mit Vorteil so durchgeführt, dass der Mitrissanteil wenigstens eines Teils der Stoffaustauschböden ≥ 10 Gew.-% beträgt. Häufig wird der Mitrissanteil wenigstens eines Teils der Stoffaustauschböden ≥ 10 bis 30 Gew-%, oder 11 bis 30 Gew.-%, oder 12 bis 30 Gew.-%, oder 13 bis 30 Gew.-%, oder 14 bis 30 Gew.-%, oder 15 bis 30 Gew.-% betragen. Als Obergrenze der genannten Bereiche kommen anstelle der 30 Gew.-% auch 28 Gew.-%, oder 25 Gew.-%, oder 20 Gew.-% in Betracht.

Erfindungsgemäß bevorzugt wird das erfindungsgemäße thermische Trennverfahren so durchgeführt, dass der Mitrissanteil wenigstens der Hälfte und besonders bevorzugt wenigstens 75 % oder aller Stofftauschböden der Trennkolonne in den vorgenannten Bereichen liegen.

Insbesondere sollten diejenigen Stoffaustauschböden in den vorgenannten Bereichen liegen, auf denen der Gehalt an (Meth)acrylmonomeren besonders hoch ist.

Dies gilt insbesondere dann, wenn die Trennkolonne als trennwirksame Einbauten ausschließlich Siebböden enthält (Zwangssiebböden und/oder Regensiebböden). Insbesondere gilt es dann, wenn die Abfolge der Siebböden beim erfindungsgemäßen Verfahren äquidistant ist.

Das Einhalten der vorgenannten Randbedingung ermöglicht es, beim erfindungsgemäßen Verfahren bei gleichzeitigem geschicktem Verkleiden problematischer Teilflächen von in der Trennkolonne enthaltenen Einbauten ganz ohne zusätzlich angebrachte Sprühdüsen für potymerisationsinhibierte Rücklaufflüssigkeit auszukommen. Selbstredend können beim erfindungsgemäßen Verfahren solche Sprühdüsen aber mitverwendet oder ausschließlich verwendet werden. Zum Beispiel dann, wenn die Trennkolonne so betrieben wird, dass der Mitrissanteil aller enthaltenen Stoffaustauschböden < 10 Gew.-% beträgt.

Als ein Polymerisationsinhibitor kann auch ein molekularen Sauerstoff enthaltendes Gas mit dem aufsteigenden Dampf durch die Trennkolonne geführt oder an den unterschiedlichsten Stellen in die Trennkolonne eingedüst werden. In einfachster Weise kann ein solches molekularen Sauerstoff enthaltendes Gas Luft sein (vgl. z.B. DE-A 10248606, DE-A 10238142 und DE-A 10217121).

Beobachtet man beim Ausüben des erfindungsgemäßen Verfahrens eine Minderung der Trennwirkung der in der Trennkolonne enthaltenen Abfolge von Stoffaustauschböden (dieser Begriff meint hier und im Folgenden insbesondere Siebböden), so kann dies dadurch ausgeglichen werden, dass man die Anzahl der Stofifaustauschböden bei gleichbleibendem Abstand (d.h., die Kolonnenhöhe) erhöht.

Anwendungstechnisch zweckmäßig sollte der Stoffaustauschbodenabstand innerhalb der Bodenabfolge sich im Bereich von 300 mm bis 900 mm bewegen. Erfindungsgemäß bevorzugt beträgt beim erfindungsgemäßen Verfahren der Bodenabstand innerhalb der Bodenabfolge 300 bis 500 mm. Im Regelfall sollte der Bodenabstand 250 mm nicht unterschreiten.

Mittels der Maßnahme der Erhöhung der Anzahl der Stoffaustauschböden ist es beim erfindungsgemäßen Verfahren möglich, den Mitrissanteil der Stoffaustauschböden auf Werte von bis zu 70 Gew.-% zu steigern, ohne die Trennwirkung nennenswert zu beeinträchtigen. D.h., die Obergrenze des Mitrissanteils wenigstens eines Teils der Stoffaustauschböden kann bei Durchführung des erfindungsgemäßen Verfahrens für die bereits genannten Bereiche anstelle der 30 Gew.-% auch 35 Gew.-%, oder 40 Gew.-% oder 50 Gew.-%, oder 60 Gew.-%, oder 70 Gew.-% betragen. Selbstredend können beim erfindungsgemäßen Verfahren auch die Mitrissanteile aller Stoffaustauschböden in diesem erweiterten Mitrissanteil liegen.

Als (Meth)acrylmonomere kommen für das erfindungsgemäße Verfahren alle diejenigen in Betracht, die eingangs dieser Schrift genannt worden sind. Das erfindungsgemäße Verfahren kann eine fraktionierende Kondensation, oder eine Rektifikation, oder eine Absorption, oder eine Strippung, oder eine Desorption sein.

Insbesondere kann das erfindungsgemäße Verfahren auf alle thermischen Verfahren der Abtrennung von (Meth)acrylmonomeren aus den eingangs dieser Schrift erwähnten Stoffgemischen angewendet werden.

Dabei kann der Gehalt der gasförmigen und/oder flüssigen Stoffgemische an
(Meth)acrylmonomeren ≥ 2 Gew.-%, oder ≥ 10 Gew.-%, oder ≥ 20 Gew.-%, oder
≥ 40 Gew.-%, oder ≥ 60 Gew.-%, oder ≥ 80 Gew.-%, oder ≥ 90 Gew.-%, oder
≥ 95 Gew.-%, oder ≥ 99 Gew.-% betragen.

Im Fall einer Anwendung von Siebböden als Stoffaustauschböden für das erfindungsgemäße Verfahren können diese wie in der DE-A 2027655, der DE-A 10156988, der DE-A 10230219, der EP-A 1029573 oder in Grundlagen der Dimensionierung von Kolonnenböden, Technische Fortschrittsberichte, Band 61, K. Hoppe, M. Mittelstrass, Verlag Theodor Steinkopff, Dresden 1967 beschrieben gestaltet werden. Dabei können die Durchtrittsöffnungen kreisförmig, elliptisch oder vieleckig gestaltet sein. Sie können auch jedwede andere Form (z.B. schlitzförmig) aufweisen. Erfindungsgemäß bevorzugt sind sie kreisförmig und in strenger Dreiecksteilung angeordnet. Beispielsweise kann der Lochdurchmesser der Siebböden (insbesondere im Fall von Dual-Flow-Böden) 5 bis 50 mm, bevorzugt 10 bis 25 mm betragen. Der Abstand zweier nächstliegender Lochmittelpunkte beträgt zweckmäßig das1,5- bis 3-fache, bevorzugt das 2- bis 2,8-fache des Lochdurchmessers, welcher über den einzelnen Siebboden bevorzugt einheitlich dimensioniert ist.

Das Öffnungsverhältnis (Verhältnis der Gesamtfläche aller Durchtrittsöffnungen des Siebbodens zur Gesamtfläche des Siebbodens multipliziert mit 100 und in %) beträgt bei erfindungsgemäß einzusetzenden Siebböden zweckmäßig 8 bis 30 % und häufig 12 bis 20 %. Die Bodendicke liegt günstig bei 1 bis 8 mm.

Erfindungsgemäße Verfahren sind z.B. Rektifikationen oder fraktionierende Kondensationen, die in Trennkolonnen durchgeführt werden, die als trennwirksame Einbauten ausschließlich Böden enthalten, von deren Anzahl wenigstens zwei, bevorzugt mehr als zwei (bevorzugt ≥ 10 %, oder ≥ 20 %, oder ≥ 30 %, oder ≥ 40 %, oder ≥ 50 %, oder ≥ 60 %, oder ≥ 75 %) und besonders bevorzugt alle Siebböden, mit besonderem Vorteil Regensiebböden mit kreisförmigen Durchtrittsöffnungen sind.

Die übrigen Böden können z.B. hydraulisch abgedichtete Querstromböden (z.B. Thormann-Böden oder Glockenböden) und/oder Ventilböden sein.

Der Gasbelastungsfaktor F der erfindungsgemäß anzuwendenden Abfolge von Siebböden liegt in der Praxis vielfach im Bereich von 1 bis 3 Pa^{0.5}, häufig im Bereich von 1,5 bis 2,5 Pa^{0.5}. Die Flüssigkeitsgeschwindigkeit liegt gleichzeitig oft im Bereich von 1 bis 50 m/h oder im Bereich von 2 bis 10 m/h.

Wie bereits erwähnt, wird das erfindungsgemäße Verfahren polymerisationsinhibiert betrieben. In der Regel werden dazu die Polymerisationsinhibitoren am Kopf der Trennkolonne in die in der Trennkolonne absteigende Flüssigphase (z.B. die Rücklaufflüssigkeit oder das Absorptionsmittel) gegeben. Als in typischer Weise erfindungsgemäß einsetzbare Polymerisationsinhibitoren seien Phenothiazin, 4-Hydroxy-2,2,6,6-tetramethylpiperidin-N-oxyl, Hydrochinon und der Monomethylether des Hydrochinons (4-Methoxyphenol) genannt. Als weitere Stabilisierungsmaßnahme kann zusätzlich, wie ebenfalls bereits beschrieben, ein molekularen Sauerstoff enthaltendes Gas, z. B. Luft, durch die Trennkolonne geführt werden. In günstigen Fällen kann auch ausschließlich mit Luft polymerisationsinhibiert werden.

Vorteilhaft werden bei erfindungsgemäß eingesetzten Regensiebböden Quervermischungen und großflächige Wellenbewegungen auf den Dual-Flow-Böden durch senkrechte flächige Einbauten, sogenannte Wellenbrecher, unterbunden. Die Wellenbrecher sind anwendungstechnisch zweckmäßig in ihrer großtechnischen Anwendung 50 bis 300 mm, vorzugsweise 150 bis 200 mm hoch, sowie 500 bis 6000 mm, vorzugsweise 1000 bis 3000 mm lang (ihre Länge kann dem Bodendurchmesser oder einem Teil des Bodendurchmessers gleich sein). Bevorzugt sitzen sie mit ihrer Unterkante nicht unmittelbar auf der Oberseite des Dual-Flow-Bodens auf, sondern sind mittels kleiner Füßchen bzw. Stege auf dem Dual-Flow-Boden so aufgesetzt, dass der Abstand ihrer Unterkannte zu Oberseite des Regensiebbodens 10 bis 60 mm, vorzugsweise 30 bis 50 mm beträgt. Die Anzahl der Stege beträgt pro Wellenbrecher 1 bis 10. Der Abstand der Wellenbrecher untereinander beträgt anwendungstechnisch zweckmäßig 100 bis 1000 mm, häufig 150 bis 500 mm. Die Flächensegmente zwischen zwei Wellenbrechern betragen normalerweise ≥ 0,2 m², jedoch meist ≤ 5 m², was die Anzahl der Wellenbrecher je Regensiebboden eingrenzt.

Vorstehende Maßnahmen eignen sich insbesondere für eine bevorzugte Ausführungsvariante der Dual-Flow-Böden von Beispiel und Vergleichsbeispiel in den Schriften DE-A 10243625 und DE-A 10247240.

Eine Erhöhung des Mitrissanteils in einer erfindungsgemäß zu betreibenden Trennkolonne ist z.B. in einfacher Weise dadurch möglich, dass ein Teil der Durchtrittsöffnungen der Stoffaustauschböden bei gleichbleibender Last abgedeckt wird.

Selbstredend kann das erfindungsgemäße Verfahren auch in Kombination mit einzelnen oder allen in den Schriften DE-A 2027655, EP-A 937488, EP-A 1044957 und EP-A 1029573 genannten, eine unerwünschte Polymerisation mindernden, Maßnahmen angewendet werden.

Ganz generell kann das erfindungsgemäße Verfahren unter Normaldruck, Überdruck oder unter reduziertem Druck durchgeführt werden.

Im besonderen eignet sich das erfindungsgemäße Verfahren für die in der DE-A 19924532, DE-A 10243625 und DE-A 10247240 beschriebenen fraktionierenden Kondensationen von Acrylsäure enthaltenden Produktgasgemischen von heterogen katalysierten Gasphasen-Partialoxidationen von C₃-Vorläufen der Acrylsäure mit molekularem Sauerstoff in Trennkolonnen, die von unten nach oben zunächst Dual-Flow-Böden und im Anschluss daran hydraulisch abgedichtete Querstrom-Stoffaustauschböden enthalten.

Das erfindungsgemäße Verfahren ist durch eine verminderte Neigung unerwünschter Poymerisatbildung charakterisiert.

Selbstredend kann auch beim erfindungsgemäßen Verfahren bei zu großen Gasbelastungsfaktoren bzw. Flüssigkeitsgeschwindigkeiten die Flüssigkeit von den Siebböden nicht mehr ausreichend abfließen, können die Stoffaustauschböden fluten. Jenseits der Flutungsgrenze ist kein sinnvoller Kolonnenbetrieb mehr möglich.

In der Regel schließen beim erfindungsgemäßen Verfahren die verwendeten Stoffaustauschböden, insbesondere verwendete Regensiebböden, bündig mit den Kolonnenwänden ab. Es gibt aber auch Ausführungsvarianten, bei denen zwischen Kolonnenwand und Boden ein Zwischenraum besteht, der nur teilweise durch Brücken unterbrochen ist. Neben den eigentlichen Durchtrittsöffnungen weisen beim erfindungsgemäßen Verfahren eingesetzte Regensiebböden allenfalls noch Öffnungen auf, die z.B. eine Befestigung des Bodens auf Auflageringen oder ähnliches ermöglichen (vgl. z.B. DE-A 10159823).

Das erfindungsgemäße Verfahren eignet sich insbesondere auch für die in der DE-A 10230219 beispielhaft beschriebene Rektifikation sowie für die in der EP-A 925272 in der Stufe (b) beschriebene Absorption.

Die erfindungsgemäßen Trennkolonnen sowie die darin befindlichen Einbauten sind in zweckmäßiger Weise aus Edelstählen (z.B. 1.4541 oder 1.4571 oder SUS 316L) gefertigt.

Zum erfindungsgemäßen Verkleiden werden zweckmäßig Edelstahlbleche desselben Typs verwendet, die verschweißt werden. Sie werden dabei möglichst dünn gewählt. Die mechanische Festigkeit muß aber gewährt sein. Zu dünne Bleche lassen sich nur schwer schweißen. Üblicherweise werden die Bleche mit einer Stärke von 0,5 bis 5 mm, vorzugsweise 1 bis 3 mm verwendet.

Werden beim erfindungsgemäßen Verfahren in die Trennkolonne separate Sprühdüsen integriert, so kann deren Anzahl aufgrund der erfindungsgemäßen Verfahrensweise beschränkt, d.h., minimiert werden.

### Beispiel und Vergleichsbeispiel

### 1. Vergleichsbeispiel

In einer Rektifikationskolonne mit 3,8 m Innendurchmesser und einer Länge von 32 m wurde ein Gemisch aufgetrennt, das die folgenden Bestandteile enthielt:

| | |
|---|---|
| 17 | Gew.-% Acrylsäure, |
| 0,02 | Gew.-% Wasser, |
| 0,0015 | Gew.-% Acrolein, |
| 0,0015 | Gew.-% Allylacrylat, |
| 0,01 | Gew.-% Furfural, |
| 0,027 | Gew.-% Essigsäure, |
| 0,2 | Gew.-% Benzaldehyd, |
| 0,003 | Gew.-% Propionsäure, |
| 0,032 | Gew.-% Maleinsäureanhydrid, |
| 58 | Gew.-% Diphyl® (Gemisch aus ca. 25 Gew.-% Diphenyl und ca. 75 Gew.-% Diphenylether), |
| 17 | Gew.-% Dimethylphthalat, |
| 3 | Gew.-% Acroloylpropionsäure, und |
| 300 | Gew.ppm Phenothiazin. |

Als trennwirksame Einbauten enthielt die Rektifikationskolonne 46 Dual-Flow-Böden, die äquidistant angeordnet waren. Der Bodenabstand betrug 400 mm. Der Zulauf des aufzutrennenden Gemischs in die Rektifikationskolonne erfolgte auf dem 8ten Boden von unten. Unterhalb des Zulaufs betrug der Durchmesser der kreisrunden Durchtrittsöffnungen in den Dual-Flow-Böden 50 mm. Oberhalb des Zulaufs betrug dieser Durchmesser 25 mm. Die Durchtrittsöffnungen waren in den Dual-Flow-Böden in strenger Dreiecksteilung angeordnet (vgl. DE-A 10230219). Das Gesamtöffnungsverhältnis (Anteil der gasdurchlässigen Fläche in der Bodenebene an der Gesamtfläche der Bodenebene) betrug unterhalb des Zulaufs 17,8 % und lag oberhalb des Zulaufs bei 12,6 %. Die Temperatur am Kopf der Kolonne betrug 80°C, der Druck 105 mbar und das Rücklaufverhältnis betrug 1,3.

Die Temperatur im Sumpf der Kolonne betrug 193°C. Der Druck oberhalb der Sumpfoberfläche betrug 230 mbar. Der Kolonnensumpf wurde mit einem Zwangsumlaufverdampfer beheizt. Der Rücklauf der Rektifikationskolonne wurde durch Zusatz von Phenothiazin so polymerisationsinhibiert, dass das Produkt, welches der Rektifikationskolonne im Seitenabzug entnommen wurde 250 Gew.ppm Phenothiazin enthielt. Zusätzlich wurden in den unteren Teil der Rektifikationskolonne zum Zweck der Polymerisationsinhibierung 600000 Nl/h Luft eingeleitet. Der Zulauf in die Rektifikationskolonne wies eine Temperatur von 152°C auf.

Am Kopf der Kolonne wurde ein Leichtsiedergemisch mit 96 Gew.-% Acrylsäure entnommen. Aus dem Sumpf der Rektifikationskolonne wurde kontinuierlich ein Schwersiedergemisch entnommen, das weniger als 0,5 Gew.-% Acrylsäure enthielt. Unterhalb des 40ten Bodens von unten wurde im Seitenabzug eine 99,6 gew.-%ige Acrylsäure als Produkt entnommen.

Zum Abstützen der Dual-Flow-Böden wurden in der Rektifikationskolonne pro Boden zwei Doppel-T-Träger eingesetzt. Der Querschenkel der T's war 120 mm breit, der beide Querschenkel verbindende Längsschenkel war 240 mm hoch. Die Stärke der Schenkel betrug 10 mm.

Nach einer Laufzeit von 40 Tagen waren die Doppel-T-Träger mit Polymerisat belegt.

### 2. Beispiel

Es wurde wie im Vergleichsbeispiel verfahren. Die Doppel-T-Träger waren jedoch gemäß Figur 2 mit Stahlblech verkleidet. Die Blechstärke betrug 2 mm. An den Rändern wurden die Bleche verschweißt. Nach einer Laufzeit von 40 Tagen waren die verkleideten Doppel-T-Träger noch immer von Polymerisat frei.

## Patentansprüche

1. Ein thermisches Trennverfahren zwischen wenigstens einem in einer eine Abfolge von Stoffaustauschböden enthaltenden Trennkolonne aufsteigenden gasförmigen und wenigstens einem in der Trennkolonne absteigenden, Polymerisationsinhibitor gelöst enthaltenden, flüssigen Stoffstrom, von denen wenigstens einer (Meth)acrylmonomere enthält und bei dem innere Oberfläche der Trennkolonne mit dem in der Trennkolonne absteigenden, Polymerisationsinhibitor gelöst enthaltenden, flüssigen Stoffstrom besprüht wird und wobei die Trennkolonne Einbauten aufweist, von denen sich wenigstens Teilflächen im Schattenbereich des versprühten absteigenden flüssigen Stoffstroms befinden, **dadurch gekennzeichnet, dass** im Schatten des versprühten absteigenden flüssigen Stoffstroms befindliche Teilflächen der Einbauten durch Verkleiden aus dem Schattenbereich herausgeführt werden.

2. Verfahren nach Anspruch 1, **dadurch gekennzeichnet, dass** innere Oberfläche der Trennkolonne **dadurch** mit dem in der Trennkolonne absteigenden flüssigen Stoffstrom besprüht wird, dass der nach oben gerichtete gasförmige Stoffstrom beim Durchtritt durch Stoffaustauschböden von der auf selbigen befindlichen Flüssigphase kleine Flüssigkeitströpfchen mitreißt und nach oben gerichtet versprüht.

3. Verfahren nach einem der Ansprüche 1 oder 2, **dadurch gekennzeichnet, dass** die Trennkolonne als Stützelemente verkleidete Doppel-T-Träger eingebaut enthält.

4. Verfahren nach einem der Ansprüche 1 oder 2, **dadurch gekennzeichnet, dass** die Trennkolonne als Stützelemente verkleidete U-förmige Träger eingebaut enthält.

5. Verfahren nach einem der Ansprüche 1 bis 4, **dadurch gekennzeichnet, dass** die Stoffaustauschböden Siebböden sind.

6. Eine Abfolge von Stoffaustauschböden enthaltende Trennkolonne, die als Stützelemente verkleidete Doppel-T-Träger und/oder verkleidete U-förmige Träger eingebaut enthält, bei der beim Betreiben eines Verfahrens nach einem der Ansprüche 1 bis 5 die im Schatten des versprühten absteigenden flüssigen Stoffstroms befindlichen Teilflächen der Doppel-T-Träger und/oder U-förmigen Träger durch die Verkleidung aus dem Schattenbereich herausgeführt werden.

## Claims

1. A thermal separating process between at least one gaseous stream ascending in a separating column comprising a sequence of mass transfer trays and a liquid stream descending in the separating column and comprising dissolved polymerization inhibitor, at least one of said streams comprising (meth)acrylic monomers, and the inner surface of the separating column being sprayed with the liquid stream descending in the separating column and comprising dissolved polymerization inhibitor, and the separating column having internals of whose surface at least parts are in the shadow region of the sprayed descending liquid stream, wherein parts of the surface of the internals which are in the shadow of the sprayed descending liquid stream are removed from the shadow region by being covered.

2. The process according to claim 1, wherein the inner surface of the separating column is sprayed with the liquid stream ascending in the separating column by the gaseous stream moving upward, as it passes through mass transfer trays, entraining small liquid droplets from the liquid phase disposed thereon and spraying them upward.

3. The process according to either of claims 1 or 2, wherein the support elements installed in the separating column are covered double-T supports.

4. The process according to either of claims 1 or 2, wherein the support elements installed in the separating column are covered U-shaped supports.

5. The process according to any of claims 1 to 4, wherein the mass transfer trays are sieve trays.

6. A separating column comprising a sequence of mass transfer trays and, installed as support elements, covered double-T supports and/or covered U-shaped supports, wherein the parts of the surface of the double-T supports and/or U-shaped supports that are in the shadow of the sprayed descending liquid stream in the course of the operation of a process according to any one of claims 1 to 5 are removed from the shadow region by the covering.

## Revendications

1. Procédé de séparation thermique entre au moins un courant de matière gazeux montant dans une colonne de séparation contenant une suite de plateaux d'échange de matière et au moins un courant de matière liquide descendant dans la colonne de séparation et contenant en solution un inhibiteur de polymérisation, parmi lesquels au moins l'un contient des monomères (méth)acryliques, procédé dans lequel une surface interne de la colonne de séparation est aspergée par le courant de matière liquide montant dans la colonne de séparation et contenant en solution un inhibiteur de polymérisation, la colonne de séparation présentant des éléments encastrés, dont au moins des surfaces partielles se trouvent dans une zone à l'abri du courant de matière liquide descendant pulvérisé, **caractérisé en ce que** les surfaces partielles, situées à l'abri du courant de matière liquide descendant pulvérisé, des éléments encastrés sont sortis de la zone à l'abri par chemisage.

2. Procédé suivant la revendication 1, **caractérisé en ce qu'**une surface interne de la colonne de séparation est aspergée par le courant de matière liquide descendant dans la colonne de séparation par le fait que le courant de matière gazeux dirigé vers le haut, lors du passage à travers des plateaux d'échange de matière, entraîne de petites gouttelettes de liquide provenant de la phase liquide située sur ceux-là même et les pulvérise en direction du haut.

3. Procédé suivant l'une des revendications 1 et 2, **caractérisé en ce que** la colonne de séparation contient sous forme encastrée des supports en double T chemisés, comme éléments de support.

4. Procédé suivant l'une des revendications 1 et 2, **caractérisé en ce que** la colonne de séparation contient à l'état encastré des supports en forme de U chemisés, comme éléments de support.

5. Procédé suivant l'une des revendications 1 à 4, **caractérisé en ce que** les plateaux d'échange de matière sont des plateaux perforés.

6. Colonne de séparation contenant une suite de plateaux d'échange de matière, qui contient, comme éléments de support, des supports en double T chemisés et/ou des supports en U chemisés à l'état encastré, et dans laquelle, lors de la mise en service d'un procédé suivant l'une des revendications 1 à 5, les surfaces partielles des supports en double T et/ou des supports en U , qui se trouvent à l'abri du courant de matière liquide descendant pulvérisé, sont sorties de la zone d'abri par le chemisage.
